(19) Europäisches Patentamt
European Patent Office
Office européen des brevets

(11) EP 3 467 495 A1

(12) EUROPEAN PATENT APPLICATION

(43) Date of publication:
10.04.2019 Bulletin 2019/15

(51) Int Cl.:
G01N 33/00 (2006.01)  G01J 3/18 (2006.01)
G01N 21/77 (2006.01)  G01N 33/24 (2006.01)

(21) Application number: 17195491.0

(22) Date of filing: 09.10.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: KWS SAAT SE
37574 Einbeck (DE)

(72) Inventors:
• BAUER, Christoph
  38678 Clausthal-Zellerfeld (DE)
• DAHL, Ludmilla
  37574 Einbeck (DE)
• GRUBER, Benjamin David
  37574 Einbeck (DE)
• SCHÜTZE, Katia
  37574 Einbeck (DE)

(54) USING FIBER BRAGG GRATING (FBG) SENSORS FOR DETERMINING SUBTERRANEAN GEOMETRICAL PLANT CHARACTERISTICS

(57) The invention relates to a method for determining subterranean geometrical characteristics of a plant or plants (P) is provided, comprising providing an optical fiber (1) having at least one fiber Bragg grating (FBG) formed therein belowground, optically interrogating the at least one FBG to measure a wavelength reflected by the at least one FBG, and determining the subterranean geometrical characteristics of the plant or plants (P) based on the measured reflected wavelength. This may provide for a nondestructive method that may allow repeated measurements over time without having to be re-installed every season. Moreover, it may be suited both for use in e.g. a glasshouse lab and in the field.

FIG. 2

## Description

### FIELD OF THE INVENTION

The invention generally relates to the determination of information about plants. More particularly, the invention relates methods and systems that make use of fiber Bragg grating (FBG) sensors for determining subterranean geometrical characteristics of a plant or plants.

### BACKGROUND OF THE INVENTION

The root system of a plant serves a number of different purposes. With its aid, both the secure anchoring in the ground and the intake of water and nutrients is realized. The "hidden half", as it is also called, is therefore a very important part of the plant that is a key driver for the plants function and yield (see Y. Waisel, A. Eshel and U. Kafkaf, Ed., "Plant Roots - The Hidden Half, Marcel Dekker, New York, 1996). It is thus desirable to harness the genetic variation in plant root systems for agricultural improvement. However, since the greater part of the roots of a plant spread belowground, their observation in general proves to be difficult and time consuming.

While some prior art methods are able to provide comprehensive information about the characteristics of an analyzed root system, these methods often require a substantive amount of work and frequently involve the destruction of the observed object.

For instance, soil coring is a destructive system of taking a soil sample and counting how many roots are present in the sample (see G. Schroth and D. Kolbe, "A method of processing soil core samples for root studies by subsampling", Biology and Fertility of Soils, Vol. 18, No. 1, June 1994, pages 60-62). This requires significant work and only gives data at a single point in time.

Minirhizotrons are image-based systems that are installed in tubes underground (see H.M. Taylor, D.R. Upchurch and B.L. McMichael, "Applications and limitations of rhizotrons and minirhizotrons for root studies", Plant and Soil, Vol. 129, No. 1, December 1990, pages 29-55). While they allow for a repeated measurement over time, they must be installed and removed every season even when measuring at depths.

Ground penetrating radar is an entirely non-destructive technique, but it is currently not suitable for small roots found in crop plants such as maize (see L. Guo et al., "Application of ground penetrating radar for coarse root detection and quantification: A review", Plant and Soil, Vol. 362, No. 1-2, January 2013, pages 1-23).

With filter paper or agar screens, plants are grown in or on an artificial medium. These methods are characterized by the complete absence of a soil-like substrate and the plants are often grown in the light.

Rhizoboxes, mesocosms and transparent pots allow growing plants in a soil or soil-like system. The roots are then either imaged along a transparent wall or they are washed out at the end of the experiment (see again H.M. Taylor, D.R. Upchurch and B.L. McMichael). These systems can produce unnatural growth conditions along the transparent wall and, if the roots are washed out, the harvest is destructive.

In the "shovelomics" method, root crowns are dug and washed out of the soil and are imaged. Again, this is a substantially destructive method.

X-ray computed tomography and magnetic resonance imaging are two non-destructive systems for imaging roots inside their natural soil environment (see R. Metzner et al., "Direct comparison of MRI and X-ray CT technologies for 3D imaging of root systems in soil: Potential and challenges for root trait quantification", Plant Methods, Vol. 11, No. 17, March 2015). They have a relatively low throughput and repeated measurements are only possible for plants in pots; for field samples, only a one-time measurement is possible.

Electrical impedance spectroscopy is a non-destructive method for quantifying root growth indirectly in the field or pot (see T. Repo, "Electrical impedance spectroscopy and roots", in "Measuring roots - An updated approach", Springer, Berlin Heidelberg, 2012). This method has the disadvantage that the roots are only indirectly quantified based on an electrical parameter that is also influenced by many soil factors. It's debated in the literature whether it is really the roots that are being measured or whether other plant characteristics, such as stem diameter, dominate the measurement result.

Root exudation measurements are often performed by microcapillary tubes (see A. Hodge, S.J. Grayston and B.G. Ord, "A novel method for characterisation and quantification of plant root exudates", Plant and Soil, Vo. 184, No. 1, March 1996, pages 97-104). Performing such measurements is not always easy and requires good access to the roots. Also, the exudation of the root is often quickly metabolized by soil microbes.

A good overview of the various known methods for plant root analysis is found in A. Paez-Garcia et al., "Root traits and phenotyping strategies for plant improvement", Plants, Vol. 4, June 2015, pages 334-355.

The described prior art methods all have different disadvantages associated with them.

### SUMMARY OF THE INVENTION

It is an object of the invention to provide a method for determining subterranean geometrical characteristics of a plant or plants, which can overcome at least some of the disadvantages of the described prior art methods. It is a further object of the invention to provide a corresponding system for determining subterranean geometrical characteristics of a plant or plants.

According to an aspect of the invention, a method for determining subterranean geometrical characteristics of a plant or plants is provided, comprising:

- providing an optical fiber having at least one fiber Bragg grating (FBG) formed therein belowground,

- optically interrogating the at least one FBG to measure a wavelength reflected by the at least one FBG, and
- determining the subterranean geometrical characteristics of the plant or plants based on the measured reflected wavelength.

As will be described in more detail later with reference to the drawings, FBG sensors can sense geometric changes (stretching or bending) of an optical fiber based on an optical interrogation of an FBG formed therein. If the optical fiber is provided belowground and the geometric changes thereof are induced by the growth of the root system of a plant or plants, the result of the optical interrogation, i.e. the measurement of a wavelength reflected by the FBG, can give information about the subterranean geometrical characteristics of the plant or plants. For instance, if the optical fiber is provided belowground at a pre-defined depth below the plant or plants, a change of the measured reflected wavelength may indicate that the root system of the plant or plants reached the pre-defined depth. Likewise, if the optical fiber is provided belowground in a suitably close proximity to the tap root of a plant, e.g. the beet of a sugar beet, a change of the measured reflected wavelength may be indicative of the three-dimensional shape and/or size of the tap root.

Compared to the described prior art methods, the use of FBG sensors for determining subterranean geometrical characteristics of a plant or plants can have a number of advantages. For instance, it can provide for a non-destructive method that may allow repeated measurements over time without having to be re-installed every season. Moreover, it may be suited both for use in e.g. a glasshouse lab and in the field.

It is preferred that the method further comprises:

- providing a temperature sensor belowground for sensing a subterranean temperature,

wherein the determining of the subterranean geometrical characteristics of the plant or plants based on the reflected wavelength corrects for the impact of the subterranean temperature on the reflected wavelength.

The wavelength reflected by the at least one FBG is not only affected by the geometry of the optical fiber but also by the temperature thereof. Thus, in order to be able to correctly attribute the result of the optical interrogation to the subterranean geometrical characteristics of the plant or plants, the impact of the subterranean temperature on the reflected wavelength is advantageously corrected.

The temperature sensor is preferably provided in close thermal contact with the optical fiber in order to ensure that the temperature it senses closely resembles the temperature of the optical fiber. In one preferred embodiment, the temperature sensor is an additional optical fiber having at least one FBG formed therein. The additional optical fiber should then preferably be provided such that it is in close thermal contact with the optical fiber but that its geometry is not affected by the growth of the root system of the plant or plants. If the at least one FBG of the optical fiber (geometry sensor) and the at least one FBG of the additional optical fiber (temperature sensor) have the same characteristics, in particular, the same Bragg wavelength, any changes of the measured reflected wavelength from the temperature sensor can directly be used for correcting the measured reflected wavelength from the geometry sensor. It can easily be seen that, in this case, it is not necessary that an actual value of the subterranean temperature or its change is determined. Rather it suffices that the impact of the subterranean temperature or its change on the measured reflected wavelength from the temperature sensor is determined to provide a reference for the geometry sensor.

It is further preferred that the optical fiber has a plurality of FBGs formed therein. As will also be described in more detail later with reference to the drawings, FBGs can be constructed with unique Bragg wavelengths. This lends itself well to so-called wavelength division multiplexing (WDM) techniques, which provide the ability to daisy chain multiple FBGs with different Bragg wavelengths along a single optical fiber over long distances. By providing the optical fiber such that it has a plurality of FBGs formed therein, it is then possible to determine the subterranean geometrical characteristics of the plant or plants at multiple locations.

It is preferred that a spacing between the plurality of FBGs formed in the optical fiber is smaller than 2.0 cm, preferably smaller than 1.0 cm, more preferably smaller than 0.5 cm, most preferably smaller than 0.2 cm. By making use of a sufficiently small spacing between the plurality of FBGs, the sampling at which the subterranean geometrical characteristics of the plant or plants are determined with the optical fiber can be comparably small.

It is preferred that the method further comprises:

- providing a concentrating structure for concentrating multiple roots of the plant or plants towards the optical fiber.

This is particularly advantageous when the plant or plants have rather fine roots of which the geometrical characteristics may not be - or at least not easily be - determined individually. In one preferred embodiment, the concentrating structure is a plate-like structure that may be provided belowground at an angle to the ground surface and the optical fiber - and possibly additional optical fibers - is/are provided in the form of a fence at a lower end of the plate-like structure. The plate-like structure may be formed, for instance, from a frame, such as a PCV frame, with longitudinal and transverse struts for stabilization, which is covered with a mesh, such as a nylon mesh. The purpose of the mesh is to prevent the roots from growing through the mesh, but still allow water to pass, and to concentrate the roots towards the fence with the optical fiber or optical fibers. The mesh opening should be adapted to the type of the plant or plants and its/their root structures. For instance, for sugar beet a mesh opening of 300 to 500 $\mu$m is sufficient whereas for maize plants a smaller mesh opening is preferable. The concentrated group of roots then grows through the fence, deforming the optical fiber or optical fibers and producing a measurable signal. The concentration of roots is helpful in order to reduce the spatial randomness of roots hitting the fibers (the chance of a single root hitting a single fiber is less than coming across the mesh and then being led to the fence of fibers). Of course, while a plate-like structure is used as the concentration structure in the described preferred embodiment, in other embodiments the concentration structure can be another kind of structure, such as a funnel-like structure, a channel-like structure et cetera.

It is further preferred that providing of the optical fiber comprises supporting the optical fiber on a support structure. This is advantageous in that it allows for a more simple installation belowground. Moreover, the use of a support structure having a pre-defined geometry for supporting the optical fiber can make it easier to associate the at least one FGB with an actual three-dimensional position belowground. In one preferred embodiment, the support structure is a substantially cylindrical structure, e.g. a paper pot structure as it is commercially available from Heto Agrotechnics, The Netherlands. Such a paper pot structure provides a growth system that is formed by rolling various types of paper or fleece into a roll in which the plant is inserted together with a growth substrate. The optical fiber - and possibly additional optical fibers - could be attached to the outside of the finished paper pot structure, but it would also be possible to attach the optical fiber or optical fibers at the inside of the paper pot structure before the insertion of the growth substrate and the plant. Still another option would be to insert the optical fiber or optical fibers between two layers of paper or fleece.

The arrangement of the optical fiber or optical fibers can be adapted according to needs. For instance, it/they could be arranged in the form of a spiral around a paper pot structure, which can allow determining the three-dimensional shape and/or size of the tap root of a plant, e.g. the beet of a sugar beet. Of course, other arrangements are also possible, wherein it must only be ensured that growth of the root system leads to a stretching or bending of the optical fiber or optical fibers.

In another preferred embodiment, the support structure is a structure that allows the optical fiber or optical fibers to be arranged in the form of a fence, as described above. For instance, the support structure may comprise two or more posts or the like on which the optical fiber or optical fibers may be attached or it may comprise a two-dimensional structure, such as a frame that can have longitudinal or traverse struts for stabilization.

It is preferred that multiple optical fibers having at least one FBG formed therein are provided at different depths belowground. With such a configuration, it can be determined how deep the roots of the plant or plants have grown at a certain point in time. Alternatively, it is also possible that a single optical fiber having a plurality of FBGs formed therein is provided at different depths belowground.

It is also preferred that the determining of the subterranean geometrical characteristics of the plant or plants based on the reflected wavelength comprises employing calibration data relating the reflected wavelength to the subterranean geometrical characteristics of the plant or plants.

This relates to the fact that the reflected wavelength itself only provides an information about the strain of the optical fiber at the at least one FBG. In order to relate this information to the subterranean geometrical characteristics of the plant or plants, it must first of all be determined by means of a suitable calibration how the growth of the tap root of a plant, e.g. the beet of a sugar beet, or the growth of the root system of a plant or plants that have the rather fine roots affects the geometry of the provided optical fiber or optical fibers. For instance, if a sugar beet is grown in a paper pot structure, as described before, the calibration on beets that are destructively harvested can provide calibration data that relate the measured reflected wavelength at a specific position of the optical fiber that is arranged in the form of a spiral around the paper pot structure to an absolute beet diameter at the specific position. Likewise, if a plate-like structure is used as a concentrating structure for concentrating multiple roots of the plant or plants towards the optical fiber, as described before, the calibration can provide calibration data that give a time delay required between the tip of a root hitting the fiber fence and the appearance of a measurable change of the reflected wavelength and that also relate the amplitude of the change of the measured reflected wavelength to the diameter of the roots (or the amount of roots).

It is preferred that the optical fiber further has at least one region that is adapted to attract and/or bind one or more substances of interest formed therein, wherein the method further comprises:

- optically interrogating the at least one region to measure spectral changes caused by the one or more substances of interest, and
- determining subterranean soil characteristics and/or the biochemical surroundings of the plant or plants based on the spectral changes.

This accounts for the fact that a number of biochemical substances in the surroundings of a plant or plants are of interest to scientists and farmers. Examples of substances of interest include all of the essential nutrients required by the plant or plants. A number of these can be quantified spectrally, providing a chance to determine the concentration in the soil under the plants. This is useful scientifically in order to know the spatial location of nutrients in the soil, and also from a management and environmental protection perspective, for example, in order to ensure that the nutrients are not lost out of the root profile. Additionally, roots exudate is a complex mixture of substances (for a comprehensive list see P.G. Dennis et al., "Are root exudates more important than other sources of rhizodeposits in structuring rhizosphere bacterial communities?", FEMS Microbiology Ecology, Vol. 72, March 2010, pages 313-327) including but not limited to mono and polysaccharides (e.g. galactose, xylose, mannose, arabinose, pectin), amino acids (e.g. proline, serine, histidine), and organic acids (e.g. malic acid, citric acid). These have a variety of functions such as maintaining contact with the soil solution during periods of drought, binding toxic elements in the soil that normally inhibit plant growth, or releasing nutrients from the soil that the plant would not normally be able to acquire. Root exudates are currently almost impossible to measure in the field under in-situ conditions. The exudates are also able to influence the microbial composition in the rhizosphere and the use of optical fibers provides a way to analyze the exudate and/or microbial composition.

The at least one region is preferably used as a kind of resonance area. This area can be doped or enhanced with nano-structures for a better sensitivity to the one or more substances of interest (e.g. constituents of root exudates). Of course, it is possible that the optical fiber has a plurality of regions that are each adapted to attract and/or bind one or more substances of interest formed therein. In that case, the one or more substances can be the same for each region or they may differ between regions, e.g. each region may be adapted to only attract and/or bind a single substance of interest. While it can be more efficient if the at least one region is formed in an optical fiber that also has at least one FBG formed therein, it is also possible that the at least one FBG and the at least one region are formed in different optical fibers. In that case, the optical fiber that has the at least one FBG formed therein can be used as a geometry sensor and the optical fiber that has the at least one region formed therein can be used as an environmental sensor.

The determining of the subterranean soil characteristics and/or the biochemical surroundings of the plant or plants based on the spectral changes preferably makes use of statistical approaches to analyze changes in the amplitude, time and/or spectral characteristics of the light that is used in the optical interrogating in order to correlate the output signal of the optical interrogating with the subterranean soil characteristics and/or the biochemical surroundings of the plant or plants.

Preferably, the method further comprises:

- determining a root disease or insect infestation of the plant or plants based on the determined biochemical surroundings of the plant or plants.

This is based on the insight that the development of root diseases or insect infestation correlates with changes in the biochemical surroundings of the plant or plants, which can be determined as described above. For instance, the growth of a fungus on a sugar beet leads to cell death with the cell contents being released (although these are often quickly consumed by microbes). The microbial population that then surrounds the dying root tissue is often associated with a biofilm of distinct chemical nature relative to a healthy growing root. The spectral signature of this biofilm can then be detected using optical fibers, thereby providing information about the distribution of the pathogen in the field and the tolerance of the genotypes without having to lift beets.

It is preferred that the optical interrogating of the at least one FBG and/or of the at least one region includes tuning the wavelength range of a tunable light source. This is particularly advantageous if the optical fiber has a plurality of FBGs and/or a plurality of regions that are adapted to attract and/or bind one or more substances of interest formed therein, since in this case the plurality of FBGs and/or regions can be optically interrogated with a single tunable light source by tuning its wavelength range of the tunable light source such that it matches the requirements for the optical interrogation of a particular FBG or region. In particular, it is possible to tune the wavelength range of the tunable light source to match the spectral region of each of the one or more substances of interest (e.g. water), respectively.

According to a further aspect of the invention, a system for determining subterranean geometrical characteristics of a plant or plants is provided, comprising:

- an optical fiber having at least one fiber Bragg grating (FBG) formed therein,
- an optical interrogation system for optically interrogating the at least one FBG to measure a wavelength reflected by the at least one FBG, and
- a determination system for determining the subterranean geometrical characteristics of the plant or plants based on the measured reflected wavelength,

wherein the system is adapted to perform the method as defined in above aspect.

It is preferred that the system further comprises:

- a concentrating structure for concentrating multiple roots of the plant or plants towards the optical fiber, and/or
- a support structure for supporting the optical fiber thereon.

It is further preferred that the optical fiber further has at least one region that is adapted to attract and/or bind one or more substances of interest formed therein,

wherein the optical interrogation system is further adapted to optically interrogate the at least one region to measure spectral changes caused by the one or more substances of interest, and
wherein the determination system is further adapted to determine subterranean soil characteristics and/or the biochemical surroundings of the plant or plants based on the spectral changes.

Preferably, the determination system is adapted such that the determining of the subterranean geometrical characteristics of the plant or plants based on the reflected wavelength comprises employing calibration data relating the reflected wavelength to the subterranean geometrical characteristics of the plant or plants.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1    shows schematically and exemplarily an optical fiber having an FBG formed therein,

Fig. 2    shows schematically and exemplarily a first embodiment of a system for determining subterranean geometrical characteristics of a plant or plants,

Fig. 3    shows schematically and exemplarily a second embodiment of a system for determining subterranean geometrical characteristics of a plant or plants,

Fig. 4    shows schematically and exemplarily a third embodiment of a system for determining subterranean geometrical characteristics of a plant or plants,

Fig. 5    shows schematically and exemplarily results of experiments in which sub-terranean geometrical characteristics of a plant or plants have been determined using optical fibers, and

Fig. 6    shows schematically and exemplarily an optical fiber having at least one region that is adapted to attract and/or bind one or more substances of interest formed therein.

DETAILED DESCRIPTION OF EMBODIMENTS

In the description, like elements or units are referred by like reference numerals. Moreover, if a particular element or unit or its function has been described with reference to a particular figure, the particular element or unit or its function may not be described repeatedly with reference to another figure in which it occurs.

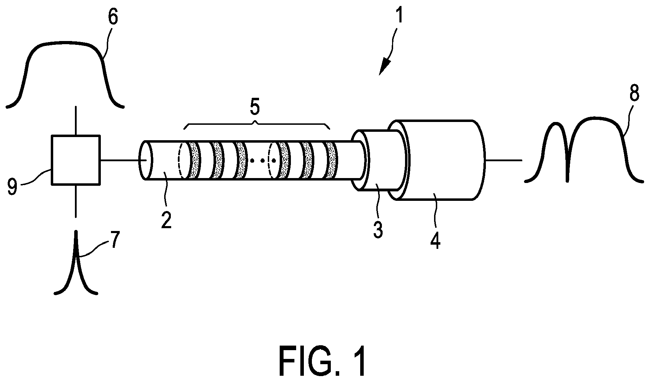

The invention concerns methods and systems that make use of fiber Bragg grating (FBG) sensors for determining subterranean geometrical characteristics of a plant. An optical fiber 1 having an FBG 5 formed therein is schematically and exemplarily shown in Fig. 1. The optical fiber 1 consists of three main components: The core 2, the cladding 3, and the buffer coating 4. The core 2 is a thin strand of glass that transmits light. It is surrounded by the cladding 3, which reflects stray light back into the core 2, ensuring the transmission of light through the core 2 with minimal loss. This is achieved with a higher reflective index in the core 2 relative to the cladding 3, causing a total internal reflection of light. The additional outer buffer coating 4 serves to protect the optical fiber from external conditions and physical damage. It can incorporate many layers depending on the amount of ruggedness and protection required.

The FBG 5 is one of the most commonly used and broadly employed optical sensors. It reflects a wavelength of light that shifts in response to variations in temperature and/or strain. FBGs are constructed by using holographic interference or a phase mask to expose a short length of photosensitive fiber to a periodic distribution of light intensity. In this way, the refractive index of the fiber is permanently altered according to the intensity of the light it is exposed to. The resulting periodic variation in the refractive index is called the "fiber Bragg grating".

The FBG 5 provides a sensing capability as follows: When a broad-spectrum light beam 6 is sent through the optical fiber 1 to the FBG 5, reflections from each segment of alternating reflective index interfere constructively only for a specific wavelength of light, which is called Bragg wavelength. This effectively causes the FBG 5 to reflect a specific frequency 7 of light which other frequencies 8 are transmitted.

The Bragg wavelength is given by:

$$\lambda_b = 2 \cdot n \cdot \Lambda,$$

where $\lambda_b$ is the Bragg wavelength, n is the effective refractive index of the fiber core 2 and $\Lambda$ is the spacing between the gratings, known as the "grating period". Because the Bragg wavelength is a function of the spacing $\Lambda$ between the gratings, FBGs can be manufactured with various Bragg wavelengths, which enables different FBGs to reflect unique wavelengths of light.

Changes in strain and temperature affect both the effective refractive index $n$ and the grating period $\Lambda$ of the FBG 5. This, in turn, results in a shift of the reflected wavelength. The change in wavelength of an FBG due to strain and temperature can be approximated by a well-known equation that comprises two expressions describing the impact of strain and the impact of temperature on the wavelength shift.

The wavelength that is reflected by the FBG 5 is determined by an optical interrogation system 9 comprising a suitable light source (not shown in the figure), for instance, a laser light source, which is coupled to the optical fiber 1. In one realization, the optical interrogation system 9 can employ a spectrometer, interferometer or other device (not shown in the figure) that can provide high-resolution optical spectrum measurements to precisely determine the reflected wavelength. Alternatively, it can involve e.g. the use of a charge-coupled device (CCD) and a fixed dispersive element (not shown in the figure) to perform a so-called wavelength-position conversion. In this latter method, the reflected light from the FBG 5 is passed though the dispersive element that distributes the wavelength components of the reflection to different locations on a linear CCD sensor.

In the following, we describe in more detail how optical fibers as shown in Fig. 1 are used according to the invention for determining subterranean geometrical characteristics of a plant or plants.

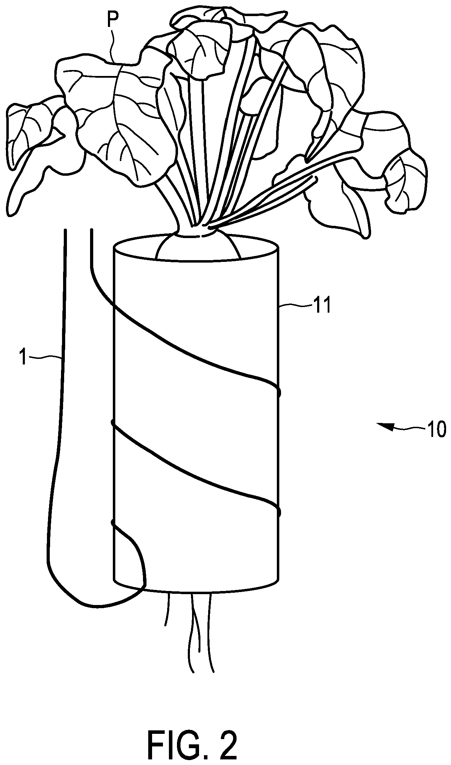

Figs. 2 to 4 show schematically and exemplarily three different embodiments of a system for determining subterranean geometrical characteristics of a plant or plants. Each of these systems is adapted to perform a method for determining subterranean geometrical characteristics of the plant or plants, comprising providing an optical fiber having at least one fiber Bragg grating (FBG) formed therein belowground, optically interrogating the at least one FBG to measure a wavelength reflected by the at least one FBG, and determining the subterranean geometrical characteristics of the plant or plants based on the measured reflected wavelength.

The system 10 shown in Fig. 2 comprises an optical fiber 1 having a plurality of FBGs formed therein, which is provided belowground. Moreover, the system 10 comprises an optical interrogation system (not shown in the figure), which is coupled to the optical fiber 1 and is adapted to optically interrogate the plurality of FBGs to measure wavelengths reflected by the plurality of FBGs, and a determination system (likewise not shown in the figure), which is adapted to determine the subterranean geometrical characteristics of the plant or plants P based on the measured reflected wavelengths. As described before, the optical interrogation system comprises a suitable light source, for instance, a laser light source, and it can determine the reflected wavelengths using, for instance, optical spectrum measurements or a wavelength-position conversion method. The determination system can be a suitably programmed processor or the like. In this embodiment, a spacing between the plurality of FBGs formed in the optical fiber 1 is smaller than 1.0 cm such that the sampling at which the subterranean geometrical characteristics of the plant or plants P are determined with the optical fiber 1 can be comparably small. In other embodiments, the spacing can even be smaller, e.g. smaller than 0.5 cm or smaller than 0.2 cm.

As can be seen from Fig. 1, the system 10 further comprises a support structure 11 on which the optical fiber 1 is supported. In this embodiment, the support structure 11 is a substantially cylindrical structure, here, a paper pot structure 11 as it is commercially available from Heto Agrotechnics, The Netherlands. Such a paper pot structure 11 provides a growth system that is formed by rolling various types of paper or fleece into a roll in which the plant P is inserted together with a growth substrate. The optical fiber 1, here, is attached to the outside of the finished paper pot structure 11 in the form of a spiral around the paper pot structure 11, but it would also be possible to attach the optical fiber 1 at the inside of the paper pot structure 11 before the insertion of the growth substrate and the plant P. Still another option would be to insert the optical fiber 1 between two layers of paper or fleece. The system 10 preferably allows determining the three-dimensional shape and/or size of the tap root of a plant P, e.g. the beet of a sugar beet.

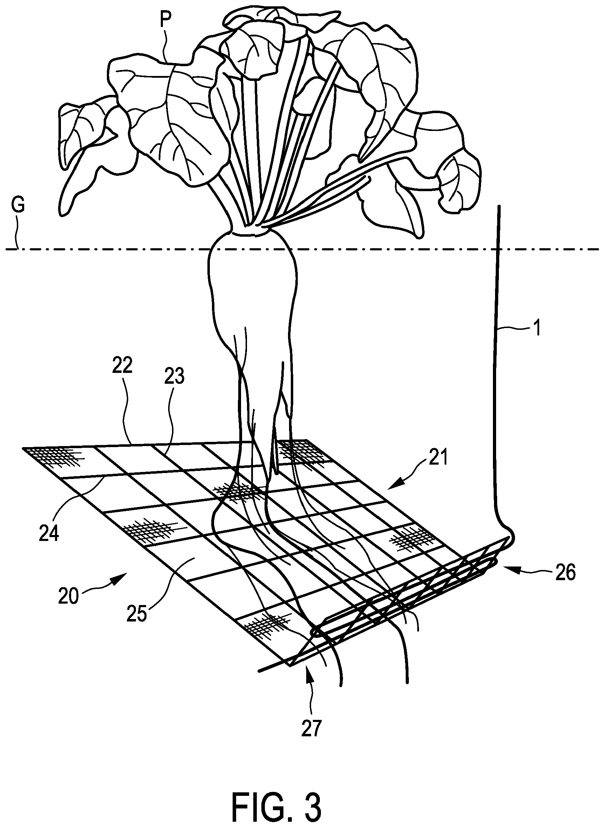

The system 20 shown in Fig. 3 also comprises an optical fiber 1 having a plurality of FBGs formed therein, which is provided belowground. Moreover, the system 20 also comprises an optical interrogation system (not shown in the figure), which is coupled to the optical fiber 1 and is adapted to optically interrogate the plurality of FBGs to measure wavelengths reflected by the plurality of FBGs, and a determination system (likewise not shown in the figure), which is adapted to determine the subterranean geometrical characteristics of the plant or plants P based on the measured reflected wavelengths. The optical fiber 1, the optical interrogation system and the determination system can be substantially the same or similar to the ones described with reference to Fig. 2 above.

As can be seen from Fig. 3, the system 20 further comprises a concentrating structure 21 for concentrating multiple roots of the plant or plants P towards the optical fiber 1. In this embodiment, the concentrating structure 21 is a plate-like structure that is provided belowground at an angle to the ground surface G and the optical fiber 1 is provided in the form of a fence 26 at a lower end of the plate-like structure 21. The plate-like structure 21 is formed, here, from a PVC frame 22 with longitudinal and transverse struts 23 and 24 for stabilization, which is covered with a nylon mesh 25. The purpose of the mesh 25 is to prevent the roots from growing through the mesh 25, but still allow water to pass, and to concentrate the roots towards the fence 26. The mesh opening is preferably adapted to the type of the pant or plants P and its/their root structures. For instance, for sugar beet a mesh opening of 300 to 500 $\mu$m is sufficient whereas for maize plants a smaller mesh opening is preferable.

As can further be seen from Fig. 3, the system 20 further comprises a support structure 27 on which the optical fiber 1 is supported. In this embodiment, the support structure 27 is a structure that allows the optical fiber 1 to be arranged in the form of a fence 26, as described above. Here, the support structure 27 comprises a two-dimensional structure, in particular, a frame that has longitudinal struts for stabilization. In this realization, the fence 26 is formed from three strands of the optical fiber 1 that are supported on the support structure 27. Each strand preferably has two, three or more FBGs 5 formed therein, preferably at the same positions in each strand.

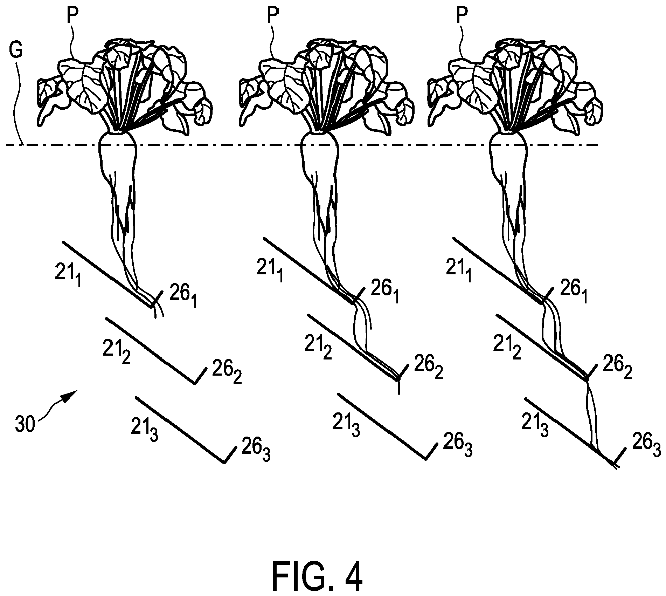

The system 30 shown in Fig. 4 is based on the system 20 shown in Fig. 3. In particular, it comprises a plurality of the concentrating structures 21, which are provided at different depths belowground at an angle to the ground surface G. In this example, three support structures $21_1$, $21_2$ and $21_3$ are provided below the plant or plants P with a spatial shift parallel to the ground surface G from one support structure to the next. On the left hand side of the figure, a situation is shown where the roots of the plant or plants P have already been grown so far that they have been concentrated by the first support structure $21_1$ and have reached the first fence $26_1$ provided at the lower end thereof. In the middle of the figure, a situation is shown where the roots of the plant or plants P have grown further, such that they have been further concentrated by the second support structure $21_2$ and have reached the second fence $26_2$ provided at the lower end thereof. Finally, on the right hand side of the figure, a situation is shown where the roots of the plant or plants P have grown even further, such that they have been further concentrated by the third support structure $21_3$ and have reached the third fence $26_3$ provided at the lower end thereof. With such a configuration, it can be determined how deep the roots of the plant or plants P have grown at a certain point in time. It shall be noted that in the system 30 shown in Fig. 4, it is possible that multiple optical fibers 1 having at least one FBG 5 formed therein are provided at the different depths belowground, i.e., each fence $26_1$, $26_2$ and $26_3$ can be formed from a separate optical fiber 1. Alternatively, it is also possible that a single optical fiber 1 having a plurality of FBGs 5 formed therein is provided at different depths belowground, e.g., the fences $26_1$, $26_2$ and $26_3$ can all be formed from the single optical fiber 1.

With respect to the systems 10, 20 and 30 shown in Figs. 2 to 4, it is noted that these preferably further comprise a temperature sensor (not shown in the figures) and is provided belowground for sensing a subterranean temperature. The determination system is the preferably adapted such that the determining of the subterranean geometrical characteristics of the plant or plants P based on the reflected wavelength corrects for the impact of the subterranean temperature on the reflected wavelength.

As described before, the wavelength reflected by the at least one FBG 5 is not only affected by the geometry of the optical fiber 1 but also by the temperature thereof. Thus, in order to be able to correctly attribute the result of the optical interrogation to the subterranean geometrical characteristics of the plant or plants P, the impact of the subterranean temperature on the reflected wavelength is advantageously corrected for.

In these embodiments, the temperature sensor is an additional optical fiber having at least one FBG formed therein, wherein the additional optical fiber is provided such that it is in close thermal contact with the optical fiber 1 but that its geometry is not affected by the growth of the root system of the plant or plants P. Here, the at least one FBG of the optical fiber 1 (geometry sensor) and the at least one FBG of the additional optical fiber (temperature sensor) have the same characteristics, in particular, the same Bragg wavelength, wherefore any changes of the measured reflected wavelength from the temperature sensor can directly be used for correcting the measured reflected wavelength from the geometry sensor.

In each of the systems 10, 20 and 30 shown in Figs. 2 to 4, the determination system is adapted such that the determining of the subterranean geometrical characteristics of the plant or plants P based on the reflected wavelength comprises employing calibration data relating the reflected wavelength to the subterranean geometrical characteristics of the plant or plants P.

As described above, this relates to the fact that the reflected wavelength itself only provides an information about the strain of the optical fiber at the at least one FBG. In order to relate this information to the subterranean geometrical characteristics of the plant or plants, it must first of all be determined by means of a suitable calibration how the growth of the tap root of a plant, e.g. the beet of a sugar beet, or the growth of the root system of a plant or plants that have the rather fine roots affects the geometry of the provided optical fiber or optical fibers. For instance, if a sugar beet is grown in a paper pot structure 11, as in the system 10 shown in Fig. 2, the calibration on beets that are destructively harvested can provide calibration data that relate the measured reflected wavelength at a specific position of the optical fiber 1 that is arranged in the form of a spiral around the paper pot structure 11 to an absolute beet diameter at the specific position. Likewise, if a plate-like structure 21 is used as a concentrating structure for concentrating multiple roots of the plant or plants P towards the optical fiber 1, as in the systems 20 and 30 shown in Figs. 3 and 4, the calibration can provide calibration data that give a time delay required between the tip of a root hitting the fiber fence 26 and the appearance of a measurable change of the reflected wavelength and that also relate the amplitude of the change of the measured reflected wavelength to the diameter of the roots (or the amount of roots).

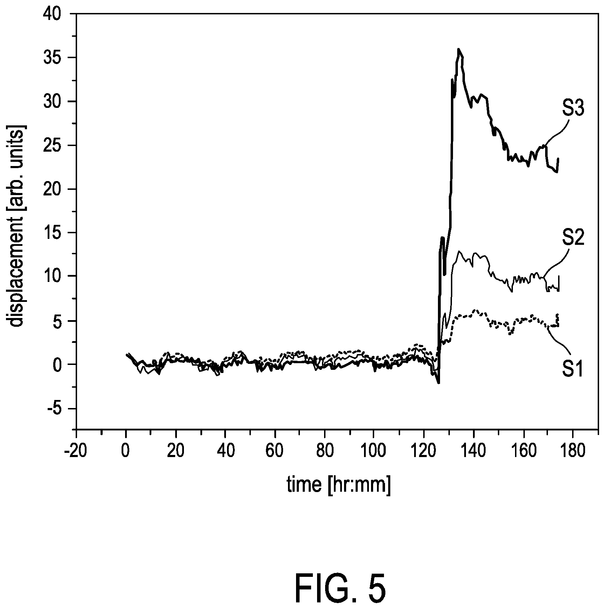

Fig. 5 shows schematically and exemplarily results of experiments in which subterranean geometrical characteristics of a plant or plants have been determined using optical fibers. In these experiments, a system like the system 20 shown in Fig. 3 was used, wherein the fence was formed from three strands of an optical fiber, each of which had eight FBGs formed therein. Over time, the roots grew along the mesh of the plate-like structure that was used as the concentration structure towards the fence and the optical fiber was displaced at three sensing positions S1, S2 and S3 (but not at sensing positions S4 to S8). The graph in Fig. 5 now shows the measurement output from the three sensing positions that produced a measureable change in the reflected wavelength (corrected for the impact of the subterranean temperature on the reflected wavelength). The increased amplitude of the measurement signals indicates the time point at that the fibers were displaced be the roots arriving at, and growing through the fence of fibers. The amplitudes represent the degree of fiber displacement (i.e. root thickness). The x-axis shows the time in hours after an arbitrary start point.

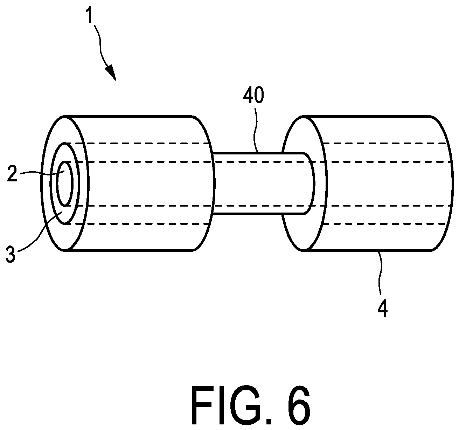

Fig. 6 shows schematically and exemplarily an optical fiber 1 having at least one region 40 that is adapted to attract and/or bind one or more substances of interest formed therein. In this case, the optical interrogation system 9 is further adapted to optically interrogate the at least one region 40 to measure spectral changes caused by the one or more substances of interest, and the determination system is further adapted to determine subterranean soil characteristics and/or the biochemical surroundings of the plant or plants P based on the spectral changes.

As described before, the at least one region 40 is preferably used as a kind of resonance area. This area can be doped or enhanced with nano-structures for a better sensitivity to the one or more substances of interest (e.g. constituents of root exudates). Of course, it is possible that the optical fiber 1 has a plurality of regions 40 that are each adapted to attract and/or bind one or more substances of interest formed therein. In that case, the one or more substances can be the same for each region 40 or they may differ between regions 40, e.g. each region 40 may be adapted to only attract and/or bind a single substance of interest. While it can be more efficient if the at least one region 40 is formed in an optical fiber 1 that also has at least one FBG 5 formed therein, it is also possible that the at least one FBG 5 and the at least one region 40 are formed in different optical fibers. In that case, the optical fiber 1 that has the at least one FBG 5 formed therein can be used as a geometry sensor and the optical fiber that has the at least one region 40 formed therein can be used as an environmental sensor.

The determining of the subterranean soil characteristics and/or the biochemical surroundings of the plant or plants P based on the spectral changes preferably makes use of statistical approaches to analyze changes in the amplitude, time and/or spectral characteristics of the light that is used in the optical interrogating in order to correlate the output signal of the optical interrogating with the subterranean soil characteristics and/or the biochemical surroundings of the plant or plants P.

Here, the determination system is further adapted to determine a root disease or insect infestation of the plant or plants based on the determined biochemical surroundings of the plant or plants.

As described before, this is based on the insight that the development of root diseases or insect infestation correlates with changes in the biochemical surroundings of the plant or plants P, which can be determined as described above. For instance, the growth of a fungus on a sugar beet leads to cell death with the cell contents being released (although these are often quickly consumed by microbes). The microbial population that then surrounds the dying root tissue is often associated with a biofilm of distinct chemical nature relative to a healthy growing root. The spectral signature of this biofilm can then be detected using optical fibers, thereby providing information about the distribution of the pathogen in the field and the tolerance of the genotypes without having to lift beets.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

Embodiments of the invention can be employed in various important applications, such as i) the monitoring of plant growth, ii) the phenotyping of the root architecture of a plant or plants, iii) the quantification of soil characteristics (e.g. humidity) and biochemical substances (e.g. exudates), iv) the monitoring of agricultural fields or field plots, and v) the phenotyping of sugar beet root rot diseases like Rhizoctonia or the phenotyping of maize roots on infestation by the European corn borer.

The invention can allow root attributes to be quantified in a non-destructive manner, a technique having application both in scientific research (i.e. root phenotyping) and in the management of agricultural systems (i.e. monitoring roots/underground processes in farmers' field). Root phenotyping has so far been technically one of the most challenging fields in plant phenotyping, but it is believed to be a very promising field, since the roots are considered the most important organ for determining the ability to take up water and nutrients. By knowing the characteristics of the root system (e.g. depth, growth rate, volume of soil explored over a certain period of time) one might be able to gain greater insights into the mechanisms of drought tolerance or nutrient efficiency, and ultimately produce cultivars excelling in these aspects.

Moreover, farmers are usually advised to keep an eye on the root systems of their crops as crops with poor root systems (due to diseases, insect attacks, soil conditions, weather, or management practices, such as fertilization) will also yield poorly or unable to cope well with stresses such as drought or disease. The invention might allow for the deployment of a system that requires a one-time installation effort, but that could potentially be used repetitively afterwards as an automated on-line measurement system requiring little farmer input. The farmer might install such a system together with the weather station on the field and log the root growth under the field as one would e.g. monitor soil moisture, allowing the farmer to respond in real-time should management practices need to be changed.

As described before, the invention relates to a method for determining subterranean geometrical characteristics of a plant or plants is provided, comprising providing an optical fiber having at least one fiber Bragg grating (FBG) formed therein belowground, optically interrogating the at least one FBG to measure a wavelength reflected by the at least one FBG, and determining the subterranean geometrical characteristics of the plant or plants based on the measured reflected wavelength. This may provide for a non-destructive method that may allow repeated measurements over time without having to be re-installed every season. Moreover, it may be suited both for use in e.g. a glasshouse lab and in the field.

**Claims**

1. A method for determining subterranean geometrical characteristics of a plant or plants (P), comprising:

   - providing an optical fiber (1) having at least one fiber Bragg grating (FBG) (5) formed therein belowground,
   - optically interrogating the at least one FBG (5) to measure a wavelength reflected by the at least one FBG (5), and
   - determining the subterranean geometrical characteristics of the plant or plants (P) based on the measured reflected wavelength.

2. The method as defined in claim 1, further comprising:

   - providing a temperature sensor belowground for sensing a subterranean temperature,

   wherein the determining of the subterranean geometrical characteristics of the plant or plants (P) based on the reflected wavelength corrects for the impact of the subterranean temperature on the reflected wavelength.

3. The method as defined in claim 1 or 2, wherein the optical fiber (1) has a plurality of FBGs (5) formed therein.

4. The method as defined in claim 3, wherein a spacing between the plurality of FBGs (5) formed in the optical fiber (1) is smaller than 2.0 cm, preferably smaller than 1.0 cm, more preferably smaller than 0.5 cm, most preferably smaller than 0.2 cm.

5. The method as defined in any of claims 1 to 4, further comprising:

   - providing a concentrating structure (21) for concentrating multiple roots of the plant or plants (P) towards the optical fiber (1).

6. The method as defined in any of claims 1 to 5, wherein the providing of the optical fiber (1) comprises supporting the optical fiber (1) on a support structure (11; 27).

7. The method as defined in any of claims 1 to 6, wherein multiple optical fibers (1) having at least one FBG (5) formed therein are provided at different depths belowground.

8. The method as defined in any of claims 1 to 7, wherein the determining of the subterranean geometrical characteristics of the plant or plants (P) based on the reflected wavelength comprises employing calibration data relating the reflected wavelength to the subterranean geometrical characteristics of the plant or plants (P).

9. The method as defined in any of claims 1 to 8, wherein the optical fiber (1) further has at least one region (40) that is adapted to attract and/or bind one or more substances of interest formed therein, wherein the method further comprises:

   - optically interrogating the at least one region (40) to measure spectral changes caused by the one or more substances of interest, and
   - determining subterranean soil characteristics and/or the biochemical surroundings of the plant or plants (P) based on the spectral changes.

10. The method as defined in claim 9, further comprising:

    - determining a root disease or insect infestation of the plant or plants (P) based on the determined biochemical surroundings of the plant or plants (P).

11. The method as defined in any of claims 1 to 10, wherein the optical interrogating of the at least one FBG (5) and/or of the at least one region (40) includes tuning the wavelength range of a tunable light source.

12. A system (10, 20, 30) for determining subterranean geometrical characteristics of a plant or plants (P), comprising:

    - an optical fiber (1) having at least one fiber Bragg grating (FBG) (5) formed therein,
    - an optical interrogation system (9) for optically interrogating the at least one FBG (5) to measure a wavelength reflected by the at least one FBG (5), and

- a determination system for determining the subterranean geometrical characteristics of the plant or plants (P) based on the measured reflected wavelength,

wherein the system (10, 20, 30) is adapted to perform the method as defined in any of claims 1 to 11.

13. The system (10, 20, 30) as defined in claim 12, further comprising:

- a concentrating structure (21) for concentrating multiple roots of the plant or plants (P) towards the optical fiber (1), and/or
- a support structure (11; 27) for supporting the optical fiber (1) thereon.

14. The system (10, 20, 30) as defined in claim 12 or 13, wherein the optical fiber (1) further has at least one region (40) that is adapted to attract and/or bind one or more substances of interest formed therein, wherein the optical interrogation system (9) is further adapted to optically interrogate the at least one region (40) to measure spectral changes caused by the one or more substances of interest, and wherein the determination system is further adapted to determine subterranean soil characteristics and/or the biochemical surroundings of the plant or plants (P) based on the spectral changes.

15. The system (10, 20, 30) as defined in any of claims 12 to 14, wherein the determination system is adapted such that the determining of the subterranean geometrical characteristics of the plant or plants (P) based on the reflected wavelength comprises employing calibration data relating the reflected wavelength to the subterranean geometrical characteristics of the plant or plants (P).

FIG. 1

FIG. 2

FIG. 3

# FIG. 4

FIG. 5

FIG. 6

Europäisches
Patentamt

European
Patent Office

Office européen
des brevets

## EUROPEAN SEARCH REPORT

Application Number

EP 17 19 5491

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 107 036 650 A (ANHUI BOTONG TRAFFIC PLANNING AND DESIGN CO LTD) 11 August 2017 (2017-08-11) * paragraph [0004] - paragraphs [0007], [0013], [0015], [0016]; figure 1 * ----- | 1-6,8,9, 11-15 | INV. G01N33/00 ADD. G01J3/18 G01N21/77 G01N33/24 |
| X | CN 106 979 791 A (SUZHOU NANZEE SENSING TECH CO LTD; UNIV NANJING) 25 July 2017 (2017-07-25) * paragraph [0002] - paragraph [0006]; figure 1 * ----- | 1,3,4,6, 8,10-12, 15 | |
| A | US 2010/259752 A1 (SHAH TUSHAR K [US] ET AL) 14 October 2010 (2010-10-14) * the whole document * ----- | 1-15 | |
| A | US 4 634 856 A (KIRKHAM RANDY R [US]) 6 January 1987 (1987-01-06) * the whole document * ----- | 1-15 | |
| A | US 4 266 878 A (AUER SIEGFRIED O) 12 May 1981 (1981-05-12) * the whole document * ----- | 1-15 | TECHNICAL FIELDS SEARCHED (IPC) G01N G01J |

The present search report has been drawn up for all claims

| Place of search | Date of completion of the search | Examiner |
|---|---|---|
| The Hague | 23 February 2018 | Pagels, Marcel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 19 5491

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2018

| Patent document cited in search report | | Publication date | Patent family member(s) | | Publication date |
|---|---|---|---|---|---|
| CN 107036650 | A | 11-08-2017 | NONE | | |
| CN 106979791 | A | 25-07-2017 | NONE | | |
| US 2010259752 | A1 | 14-10-2010 | US 2010259752 A1<br>WO 2010120729 A1 | | 14-10-2010<br>21-10-2010 |
| US 4634856 | A | 06-01-1987 | NONE | | |
| US 4266878 | A | 12-05-1981 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Plant Roots - The Hidden Half. Marcel Dekker, 1996
- **G. SCHROTH ; D. KOLBE.** A method of processing soil core samples for root studies by subsampling. *Biology and Fertility of Soils,* June 1994, vol. 18 (1), 60-62
- **H.M. TAYLOR ; D.R. UPCHURCH ; B.L. MC-MICHAEL.** Applications and limitations of rhizotrons and minirhizotrons for root studies. *Plant and Soil,* December 1990, vol. 129 (1), 29-55
- **L. GUO et al.** Application of ground penetrating radar for coarse root detection and quantification: A review. *Plant and Soil,* January 2013, vol. 362 (1-2), 1-23
- **R. METZNER et al.** Direct comparison of MRI and X-ray CT technologies for 3D imaging of root systems in soil: Potential and challenges for root trait quantification. *Plant Methods,* March 2015, vol. 11 (17

- Electrical impedance spectroscopy and roots. **T. RE-PO.** Measuring roots - An updated approach. Springer, 2012
- **A. HODGE ; S.J. GRAYSTON ; B.G. ORD.** A novel method for characterisation and quantification of plant root exudates. *Plant and Soil,* March 1996, vol. 184 (1), 97-104
- **A. PAEZ-GARCIA et al.** Root traits and phenotyping strategies for plant improvement. *Plants,* June 2015, vol. 4, 334-355
- **P.G. DENNIS et al.** Are root exudates more important than other sources of rhizodeposits in structuring rhizosphere bacterial communities?. *FEMS Microbiology Ecology,* March 2010, vol. 72, 313-327